# EUROPEAN PATENT APPLICATION

(11) **EP 4 772 630 A1**
(43) Date of publication of application: **08.07.2026**
(21) Application number: 24858668.7
(22) Date of filing: 29.08.2024
(51) Int. Cl.: C12N 15/11, C12N 15/113, A61K 31/7088, A61K 31/7105, A61P 31/00, A61P 35/00

(54) **RNA-TARGETING COMPOSITION AND USE THEREOF**

(30) Priority: 01.09.2023 CN 202311137198
(71) Applicant: The Chinese University of Hong Kong, Shenzhen, Shenzhen, Guangdong 518000 (CN); The Chinese University of Hong Kong, Shenzhen Futian Biomedical Innovation R & D Center, Shenzhen, Guangdong 518000 (CN)
(72) Inventor: CHEN, Gang, Shenzhen, Guangdong 518000 (CN); LU, Rongguang, Shenzhen, Guangdong 518000 (CN); DENG, Liping, Shenzhen, Guangdong 518000 (CN); LIAN, Yun, Shenzhen, Guangdong 518000 (CN); ZHAN, Xuan, Shenzhen, Guangdong 518000 (CN); YANG, Lixia, Shenzhen, Guangdong 518000 (CN); LIN, Ruiyu, Shenzhen, Guangdong 518000 (CN); FAN, Shijian, Shenzhen, Guangdong 518000 (CN)
(74) Representative: Metida
(86) International application number: PCT/CN2024/115426
(87) International publication number: WO 2025/045136

(57) **Abstract**

A composition includes a first RNA ligand and a second RNA ligand. The first RNA ligand comprises an oligonucleotide capable of specifically targeting a double-stranded segment of a target RNA element, and the second RNA ligand comprises an oligonucleotide capable of specifically targeting a single-stranded segment of the target RNA element, such that the composition can specifically target the double strand-single strand junction region of the target RNA element. The composition of the present application is used to target complex RNA structures and is expected to become a third-generation novel nucleic acid ligand with tremendous application prospects.

## Description

This application claims priority to the Chinese patent application No. 202311137198.3, filed with the China National Intellectual Property Administration on September 1, 2023, entitled "RNA-targeting Composition and Use Thereof," the entire content of which is incorporated herein by reference.

### TECHNICAL FIELD

The present application relates to the field of biomedicine, and particularly relates to compositions targeting RNA. The present application further relates to the use of the compositions for regulating the function of RNA elements in vivo and/or regulating the expression of RNA elements in vivo, and the use of the compositions for the prevention and/or treatment of diseases.

### BACKGROUND

RNA molecules play vital roles throughout biological processes. The self-folding secondary and tertiary structures of RNA fundamentally regulate the encoding, decoding, splicing, and expression of genes. Recent advances in the study of RNA structure, folding thermodynamics, kinetics, and functions have provided a foundation for developing nucleic acid-based diagnostics, therapeutics, and biotechnologies.

A variety of RNA molecules play important regulatory roles in both healthy and diseased cells. Although only 1-2% of the human genome encodes proteins, it is now known that the majority of the genome is transcribed (Caminci et al., Science 309:1559-1563; 2005). Thus, non-coding transcripts (the non-coding transcriptome) represent a large new class of therapeutic targets. Non-coding RNAs, such as microRNA (miRNA) and long non-coding RNA (lncRNA), regulate transcription, splicing, mRNA stability/decay, and translation. Furthermore, non-coding regions of mRNA, such as the 5' untranslated region (5' UTR), 3' UTR, and introns, can play regulatory roles in affecting mRNA expression levels, alternative splicing, translation efficiency, and the subcellular localization of mRNA and proteins. RNA secondary and tertiary structures are critical for these regulatory activities.

Notably, GWAS studies show that there are significantly more single nucleotide polymorphisms (SNPs) associated with human diseases in the non-coding transcriptome relative to coding transcripts (Maurano et al., Science 337:1190-1195; 2012). Therefore, therapeutic targeting of non-coding RNA and non-coding regions of mRNA can yield novel agents for treating previously refractory human diseases.

Current therapeutic methods for blocking mRNA require approaches such as gene therapy, genome editing, or a broad range of oligonucleotide technologies (antisense, RNAi, etc.). Oligonucleotides regulate the action of RNA through canonical base/base hybridization. The appeal of this approach is that the basic pharmacophore of the oligonucleotide can be defined in a straightforward manner by the sequence to be blocked. Many RNA ligands, such as peptide nucleic acids (PNA), antisense oligonucleotides, and interfering RNAs, have been applied in therapy.

PNA is a synthetic nucleotide analog consisting of a polypeptide chain of amino acid residues and nucleotides linked by phosphodiester bonds. The backbone of PNA is neutral and is not recognized by proteases or nucleases, exhibiting high stability. Traditional antisense PNA (asPNA) can interfere with the function of corresponding RNA by forming a PNA-RNA duplex through reverse complementarity with the corresponding RNA sequence.

The major groove of a double-stranded RNA (dsRNA) structure can form a triplex with a third strand (triplex-forming oligonucleotide, TFO) via base pairing. Thus, triplex-forming strategies are expected to be applied in developing programmable ligands that bind tightly and specifically to RNA structures. However, due to electrostatic repulsion, triplex structures formed by traditional TFOs with negatively charged phosphate backbones are typically unstable, and the formation of C+·G-C base triplexes is strongly pH-dependent. Additionally, natural bases C and T can recognize G-C and A-U base pairs, respectively, but no natural bases can recognize C-G and U-A base pairs. Nucleic acid chemists have performed extensive chemical modifications on sugar-phosphate backbones and bases to improve the properties of TFOs. Existing technologies focus on designing dbPNA (double-stranded RNA binding PNA) oligomers to target stable RNA secondary structures and form dbPNA-RNA2 triplexes therewith, thereby affecting the original RNA tertiary structure or protein binding and interfering with its function.

However, many RNA structures in vivo are simultaneously composed of dsRNA and single-stranded RNA (ssRNA) to form complex RNA tertiary structures, or RNA double-stranded regions are unwound by proteins with helicase activity during life processes, forming relatively complex dsRNA-ssRNA regions. This renders dbPNAs, which specifically bind to dsRNA, inapplicable for such designs. In addition, traditional asPNAs require complete base pairing with the corresponding RNA to bind; however, when facing complex RNA tertiary structures, they cannot fully invade and open the structures, resulting in weak binding affinity between the asPNA and the corresponding sequence, which severely limits the application of asPNA.

Therefore, there is a current need to develop nucleic acid drugs with improved binding capabilities.

### SUMMARY

Accordingly, it is an object of the present application to provide, based on the prior art, a composition capable of binding to both double-stranded RNA and single-stranded RNA. The composition of the present application can specifically target the double strand-single strand junction region of a target RNA, exhibiting better binding affinity for complex RNA structures, and thus possesses broad application prospects.

The object of the present application is achieved through the following technical solutions:
A first aspect of the present application provides a composition comprising a first RNA ligand and a second RNA ligand; wherein the first RNA ligand comprises an oligonucleotide capable of specifically targeting a double-stranded segment of a target RNA element, and the second RNA ligand comprises an oligonucleotide capable of specifically targeting a single-stranded segment of the target RNA element, such that the composition can specifically target a double strand-single strand junction region of the target RNA element.

In a preferred embodiment, the first RNA ligand forms a triplex with the double-stranded segment in the RNA structure through non-Watson-Crick pairing.

In a preferred embodiment, the second RNA ligand forms a duplex with the single-stranded segment in the RNA structure through Watson-Crick pairing.

In a preferred embodiment, the composition can specifically target the double strand-single strand junction region of the target RNA element, thereby overcoming the defect that nucleic acid drugs of the prior art cannot target complex RNA structures.

In a preferred embodiment, the first RNA ligand comprises an oligonucleotide comprising a chemical modification, wherein the chemical modification results in a selective binding of the first RNA ligand to a double-stranded RNA; and/or
the chemical modification results in an attenuation of binding between the first RNA ligand and single-stranded RNA.

In a preferred embodiment, the first RNA ligand and/or the second RNA ligand comprise at least one nucleic acid selected from the following:
a peptide nucleic acid (PNA), a ribonucleic acid (RNA), a locked nucleic acid (LNA), a phosphorodiamidate morpholino oligomer (PMO), a glycol nucleic acid (GNA), a threose nucleic acid (TNA), and a bridged nucleic acid (BNA).
the first RNA ligand and/or the second RNA ligand is connectable to functional molecule having different functions; and
preferably, the functional molecules are one or more selected from the group consisting of a fluorescent molecule, a binding-induced emission molecule, a PET imaging probe, a cell-penetrating small molecule, a targeted delivery molecule, a nanocarrier, an additional RNA ligand, a nanobody, and/or a protein binding ligand.

In a preferred embodiment, the first RNA ligand comprises a chemically modified peptide nucleic acid; and/or
the second RNA ligand comprises an antisense peptide nucleic acid.

In a preferred embodiment, the first RNA ligand comprises no less than 3 bases; and/or
the second RNA ligand comprises no less than 3 bases; and/or,
the composition comprises no more than 20 bases; and/or
a linker or no linker is included between the first RNA ligand and the second RNA ligand;
preferably, the linker is one or more aminoacetic acids or diaminobutyric acids.

In a preferred embodiment, the peptide nucleic acid comprising the chemical modification are selected from one or more of the peptide nucleic acids as follows:
i) a peptide nucleic acid forming a triplex with a G-C base pair, as shown below: 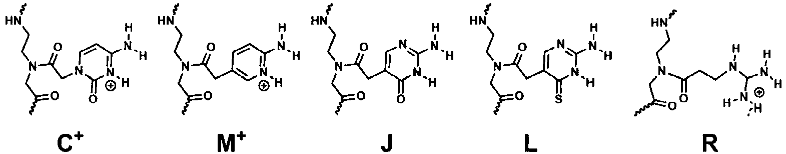 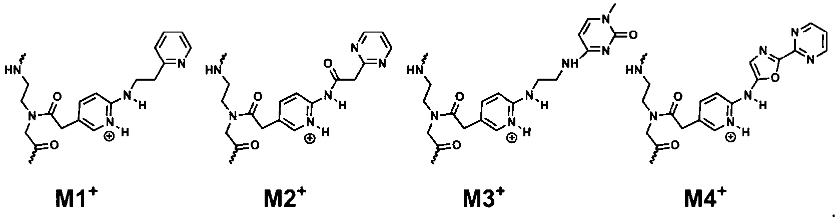
ii) a peptide nucleic acid forming a triplex with an A-U base pair, as shown below: 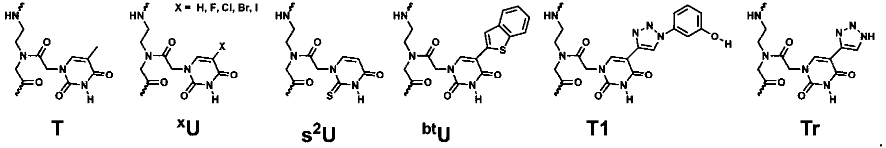
iii) a peptide nucleic acid forming a triplex with a C-G base pair, as shown below: and 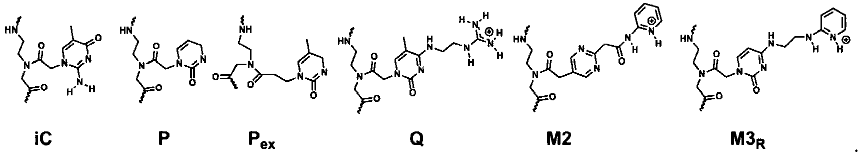
iv) a peptide nucleic acid forming a triplex with a U-A base pair, as shown below:

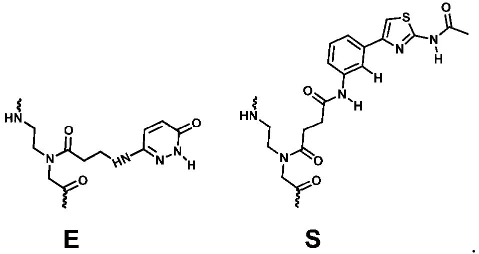

In a preferred embodiment, the target RNA comprises one or more selected from a group consisting of a nuclear RNA, a cytoplasmic RNA, a membrane RNA, and an extracellular RNA, and the target RNA element comprises a double-stranded segment and a single-stranded segment; and
preferably, the nuclear RNA or the cytoplasmic RNA is selected from a non-coding RNA, an mRNA, a miRNA, an enhancer RNA, a nascent RNA, a chromosome-enriched RNA, a ribosomal RNA, a membrane-enriched RNA, or a mitochondrial RNA.

In a preferred embodiment, the target RNA element is associated with a disease or a disorder.

In a preferred embodiment, the target RNA element comprises an RNA sequence selected from a miRNA, a novel coronavirus, or a human immunodeficiency virus HIV.

In a preferred embodiment, the novel coronavirus comprises a pseudoknot structure of a novel coronavirus SARS-CoV-2.

In a preferred embodiment, the miRNA is miRNA-21.

In a preferred embodiment, the composition is a peptide nucleic acid oligomer targeting the double strand-single strand junction region of the SARS-CoV-2 pseudoknot structure, and has the following structure, wherein the bases TTL are the first RNA ligand, and the bases ACGGGC2 are the second RNA ligand (wherein 2 is s²U): 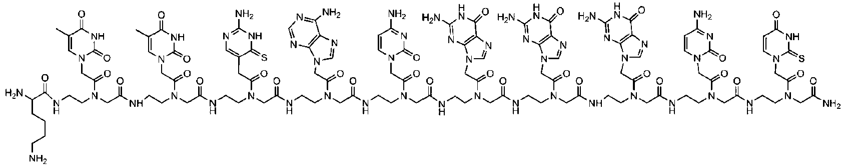 NH₂-Lys-TTLACGGGC2-CONH₂

In a preferred embodiment, the composition is a composition targeting a double strand-single strand junction region of miRNA-21, and has the following structures, wherein the bases TQTTQT are the first RNA ligand, and the bases CAACAG are the second RNA ligand: 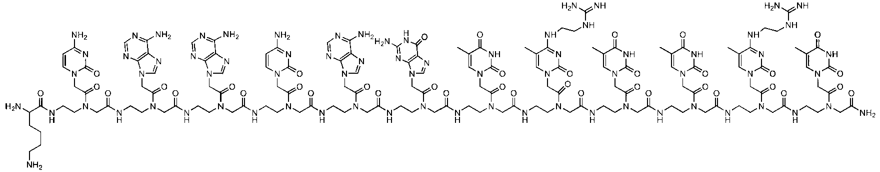 NH₂-Lys-CAACAGTQTTQT-CONH₂.

A second aspect of the present application provides a nucleic acid drug, wherein the nucleic acid drug comprises the aforementioned composition.

A third aspect of the present application provides a pharmaceutical composition, wherein the pharmaceutical composition comprises the aforementioned composition, or the aforementioned nucleic acid drug, and a pharmaceutically acceptable carrier.

A fourth aspect of the present application provides the use of the aforementioned composition, the aforementioned nucleic acid drug, or the aforementioned pharmaceutical composition in manufacture of a reagent for the regulation of the function of an RNA element in a biological organism, wherein:
after the composition, the nucleic acid drug, or the pharmaceutical composition is contacted with an organism, a structure and/or an expression of the RNA element is changed compared to that observed under reference conditions, thereby resulting in a change in a biological function of the RNA element; and
the reference conditions are selected from: an absence of the composition, a presence of a reference composition, and combinations thereof.

A fourth aspect of the present application provides the use of the aforementioned composition, the aforementioned nucleic acid drug, or the aforementioned pharmaceutical composition in manufacture of a medicament for treating a disease;
wherein the disease is associated with a functional change of an RNA element.

In some preferred embodiments, after the composition, the nucleic acid drug, or the pharmaceutical composition is contacted with an organism, a structure and/or an expression of the RNA element is changed compared to that observed under reference conditions, thereby resulting in a change in a biological function of the RNA element;
the reference conditions are selected from: an absence of the composition, a presence of a reference composition, and combinations thereof.

In some preferred embodiments, the disease is selected from cancer; in some preferred embodiments, the disease is selected from neurodegenerative diseases; in some preferred embodiments, the disease is selected from metabolic disorders; in some preferred embodiments, the disease is selected from inflammatory disorders; in some preferred embodiments, the disease is selected from autoimmune diseases; in some preferred embodiments, the disease is selected from infectious diseases; and in some preferred embodiments, the disease is selected from genetic diseases.

A fifth aspect of the present application provides a method for the modulation of the function of an RNA element and/or the regulation of the expression of an RNA element in a biological organism, wherein the method comprises contacting the organism with the aforementioned composition, the aforementioned nucleic acid drug, or the aforementioned pharmaceutical composition, wherein:
after the composition, the nucleic acid drug, or the pharmaceutical composition is contacted with an organism, a structure and/or an expression of the RNA element is changed compared to that observed under reference conditions, thereby resulting in a change in a biological function of the RNA element; and
the reference conditions are selected from: the absence of the composition, the presence of a reference composition, and combinations thereof.

A sixth aspect of the present application provides a method for the treatment of a disease in a subject, wherein the method comprises administering a therapeutically effective amount of the aforementioned composition, the aforementioned nucleic acid drug, or the aforementioned pharmaceutical composition to a subject in need thereof.

In some preferred embodiments, the disease is selected from cancer; in some preferred embodiments, the disease is selected from neurodegenerative diseases; in some preferred embodiments, the disease is selected from metabolic disorders; in some preferred embodiments, the disease is selected from inflammatory disorders; in some preferred embodiments, the disease is selected from autoimmune diseases; in some preferred embodiments, the disease is selected from infectious diseases; and in some preferred embodiments, the disease is selected from genetic diseases.

Prior art focuses on the study of dbPNA-RNA₂ triplexes. On this basis, the present application provides the composition, wherein the first RNA ligand of the composition of the present application forms a triplex with the double-stranded region in the RNA structure through non-Watson-Crick pairing, and the first RNA ligand is capable of forming a duplex with the single-stranded region through Watson-Crick pairing, which greatly enhances the application of the composition of the present application in the targeting of RNA functional elements. More importantly, by the compensation for the disadvantages of dbPNA and asPNA and the organic combination of the advantages of both, the composition of the present application is expected to become a third-generation novel nucleic acid ligand with great application prospects for the targeting of complex RNA structures.

The present application is illustrated by the accompanying drawings and the examples. The examples are not limitations on the present application.

### BRIEF DESCRIPTION OF DRAWINGS

To describe the technical solutions of the present application more clearly, the technical solutions of the present application will be described in detail below in conjunction with the accompanying drawings:
FIG. 1 is a schematic diagram illustrating the binding of a modified peptide nucleic acid to a paired RNA base according to the present application.
FIG. 2 illustrates the compositions daPNA H1S2 and dbPNA P5 designed based on the rHP1-5t RNA hairpin structure, and the binding results of said compositions with the rHP1-5t hairpin structure and variants thereof, according to an embodiment of the present application;
   wherein (a) shows daPNA H1S2 and dbPNA P5 designed based on rHP1-5t; (b) shows the RNA hairpin structure (5t-Cy3); (c) shows the binding of the RNA hairpin to the PNA; and (d) shows non-denaturing PAGE data;
   wherein the gel shown is a representative gel; and errors given are standard errors.
FIG. 3 illustrates the stimulation of programmed ribosomal frameshifting (PRF) efficiency using the composition daPNA H1S2 binding with the rHP1-5t complex according to an embodiment of the present application;
   (a) shows daPNA H1S2, dbPNA P5, and NC designed based on rHP1-5t and their binding to RNA; (b) shows the PRF efficiency of daPNA-H1S2 binding to rHP1-5t.
FIG. 4 illustrates the results of mCherry and eGFP fluorescence intensities of a dual-fluorescence protein reporter system using the composition daPNA H1S2 binding with the rHP1-5t complex according to an embodiment of the present application.
FIG. 5 illustrates a composition daPNA-SFSE-1E-2 targeting a SARS-CoV-2 pseudoknot structure and the results of its regulation of frameshifting efficiency, according to another embodiment of the present application;
   wherein (a) is a schematic diagram of the designed daPNA binding to the SARS-CoV-2 pseudoknot structure; (b) the results of an extracellular regulation of ribosomal frameshifting efficiency by the daPNAs; and (c) an antiviral effect of the composition daPNA-SFSE-1E-2 targeting the SARS-CoV-2 pseudoknot structure;
   wherein the mRNA copy number is calculated according to the standard curve formula (Y=-3.7823X+44.339) based on CT values; significance tests are performed in comparison with a liposome control group; ns P > 0.05;* P < 0.1;* * P < 0.01;* * * P < 0.001; daPNA-SFSE-1E-2 can inhibit viral replication in intracellular experiments, with an effective concentration ranging from 0.1 to 20 µM.

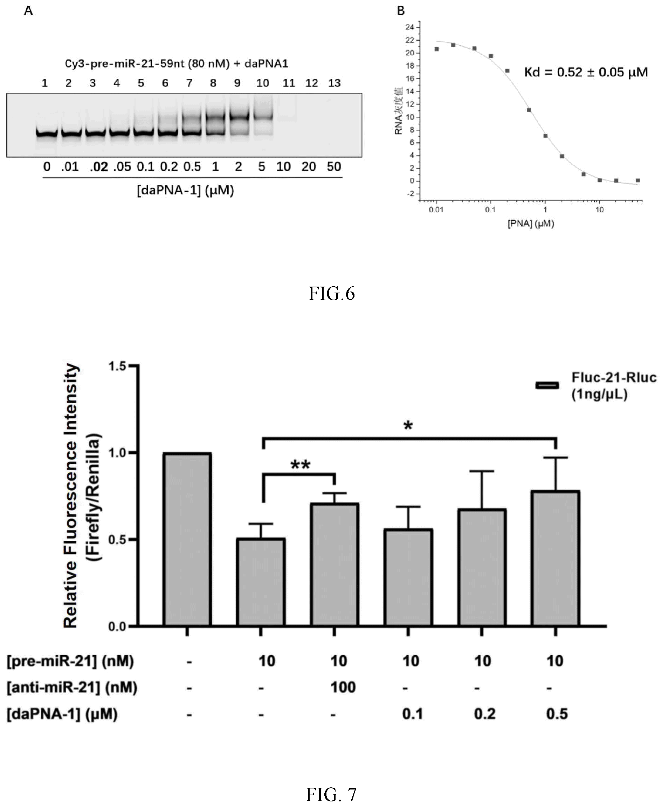

FIG. 6 illustrates a composition targeting a microRNA (miRNA) precursor can inhibit biological functions, according to another embodiment of the present application;
   wherein (a) non-denaturing polyacrylamide gel electrophoresis (PAGE) shows that daPNA-1 has high binding affinity for Cy3-pre-miR-21-59nt; (b) the Kd value of daPNA-1 binding to the miRNA precursor (Cy3-pre-miR-21-59nt) is 0.52+0.05µM;
   incubation buffer: 200 mM NaCl, 0.5 mM EDTA, 20 mM HEPES, pH 7.5; the concentration of Cy3-pre-miR-21-59nt in all samples is 80 nM; Cy3-pre-miR-21-59nt forms a hairpin structure via snap-cooling, is then slowly cooled with PNA from 65°C to room temperature, and is incubated at 4°C overnight.
FIG. 7 illustrates the dual-luciferase assay results of the miRNA-21 precursor and daPNA-1 according to another embodiment of the present application, showing that daPNA-1 can inhibit the biological function of miRNA-21 in vivo;
   wherein the full-length miRNA-21 precursor (pre-miR-21-59nt) and daPNA-1 are co-transfected into HEK-293T cells (using anti-miR-21 as a positive control); P-values are calculated using a t-test; a P-value < 0.05 is considered statistically significant.

### DESCRIPTION OF EMBODIMENTS

The technical solutions of the present application are described below with reference to specific experimental examples, but the scope of protection of the present application is not limited thereto.

The content described in the experimental examples of this specification is merely an enumeration of the forms of implementation of the inventive concept. The scope of protection of the present application should not be regarded as being limited to the specific forms set forth in the experimental examples. The scope of protection of the present application also encompasses equivalent technical means that can be conceived by those skilled in the art based on the concept of the present application. Although the embodiments of the present application are described below, the present application is not limited to the aforementioned specific embodiments and application fields. The following specific embodiments are merely illustrative and guiding, rather than restrictive. Based on the enlightenment of this specification, those of ordinary skill in the art can also make many forms without departing from the scope protected by the claims of the present application, all of which fall within the protection of the present application.

The practice of the present application involves the use of many conventional techniques in molecular biology. These techniques are well known and described, for example, in "Current Protocols in Molecular Biology", Volumes I, II, and III, 1997 (edited by F.M. Ausubel); Sambrook et al., 1989, "Molecular Cloning: A Laboratory Manual", Second Edition, Cold Spring Harbor Laboratory Press, Cold Spring Harbor, N.Y.; "DNA Cloning: A Practical Approach", Volumes I and II, 1985 (edited by D.N. Glover); "Oligonucleotide Synthesis", 1984 (edited by M.L. Gait); "Nucleic Acid Hybridization", 1985 (Hames and Higgins); "Transcription and Translation", 1984 (edited by Hames and Higgins); "Animal Cell Culture", 1986 (edited by R.I. Freshney); "Immobilized Cells and Enzymes", 1986 (IRL Press); Perbal, 1984, "A Practical Guide to Molecular Cloning"; "Methods in Enzymology" series (Academic Press, Inc.); "Gene Transfer Vectors for Mammalian Cells", 1987 (edited by J.H. Miller and M.P. Calos, Cold Spring Harbor Laboratory); and "Methods in Enzymology", Volumes 154 and 155 (edited by Wu and Grossman and Wu, respectively).
PNA: peptide nucleic acid
asPNA: antisense peptide nucleic acid
dbPNA: double-strand binding peptide nucleic acid
daPNA: double-single strand binding peptide nucleic acid
dsRNA: double-stranded RNA
ssRNA: single-stranded RNA
miRNA: miRNA
TFO: triplex-forming oligonucleotide
SARS-CoV-2: novel coronavirus
daPNA H1S2: a composition H1S2 designed based on rHP1-5t targeting a double-single strand junction region thereof
dbPNA P5: a composition P5 designed based on rHP1-5t targeting a double strand thereof
daPNA-SFSE-1E-2: a daPNA oligomer designed based on the SARS-CoV-2 RNA sequence targeting stem-1 and stem-2 of the SARS-CoV-2 RNA pseudoknot structure
rHPl-5t-Cy3: rHP1-5t RNA with Cy3 labeling
rHP1-5t: RNA hairpin structure with a partial sequence at the 5' end truncated
rHP1-5m: RNA hairpin structure with a partial sequence at the 5' end mutated
rHP1-5t-U29C-Cy3: rHP1-5t with a mutation of the base at position 29 to C
pre-miR-21-59nt: Precursor microRNA-21, where 59nt is the sequence length
Cy3-pre-miR-21-59nt: precursor microRNA-21 with Cy3 labeling
NC: negative control with no binding
mCherry: red fluorescent protein
eGFP: enhanced green fluorescent protein
dbPNA: double-stranded RNA binding peptide nucleic acid
PRF: Programmed -1 Ribosomal Frameshifting
K_{D}: Binding affinity parameter
Target RNA - mRNA

Within an mRNA, non-coding regions can affect the levels of mRNA and protein expression. In simple terms, these non-coding regions comprise IRES and upstream open reading frames (uORF) that affect translation efficiency; intron sequences that affect splicing efficiency and alternative splicing patterns; 3'UTR sequences that affect mRNA and protein localization; and elements that control mRNA decay and half-life. The therapeutic regulation of these RNA elements can have beneficial effects.

The expression of an mRNA and its translation products may be affected by targeted non-coding sequences and structures in the 5' and 3' UTRs. For example, RNA structures in the 5'UTR can affect translation efficiency. RNA structures in the 5'UTR, such as hairpins, have been confirmed to affect translation. Generally, RNA structures are considered to play a key role in the translation of mRNA. Two examples of these RNA structures are the internal ribosome entry site (IRES) and the upstream open reading frames (uORF), which can affect the translation levels of a main open reading frame. For example, nearly half of all human mRNAs have uORFs, and many of them reduce translation of the main ORF. The compositions of the present application targeting these RNAs can be used to regulate specific protein levels to obtain therapeutic benefits.

### Target RNA - Non-coding RNA

The largest group of RNA targets comprises RNAs that are transcribed but not translated into proteins, referred to as "non-coding RNAs". Non-coding RNAs are highly conserved, and many types of non-coding RNAs perform a wide range of regulatory functions. As used herein, the term "non-coding RNA" includes, but is not limited to, microRNA (miRNA), long non-coding RNA (lncRNA), long intergenic non-coding RNA (lincRNA), Piwi-interacting RNA (piRNA), competing endogenous RNA (ceRNA), and pseudogenes. These subcategories of non-coding RNAs provide a large number of RNA targets with significant therapeutic potential. Accordingly, in some embodiments, the present application provides methods of treating a disorder mediated by non-coding RNAs. In some embodiments, the disease is caused by miRNA, lncRNA, lincRNA, piRNA, ceRNA, or a pseudogene. In another aspect, the present application provides a method of producing the compositions of the present application that regulate an activity of a target RNA - non-coding RNA to treat a disease or disorder, comprising the following steps: screening one or more of the disclosed compositions that bind with said target RNA - non-coding RNA; and analyzing results by an RNA binding assay disclosed herein. In some embodiments, the target RNA - non-coding RNA is miRNA, lncRNA, lincRNA, piRNA, ceRNA, or a pseudogene.

miRNAs are short double-stranded RNAs that regulate gene expression. Each miRNA can affect the expression of multiple human genes. There are nearly 2,000 types of miRNAs in the human body. These RNAs regulate many biological processes, including cell differentiation, cell fate, movement, survival, and function. The levels of miRNA expression differ between different tissues, cell types, and disease contexts. Compared with normal tissues, they are often abnormally expressed in tumors, and their activities can play an important role in cancer. miRNAs have been confirmed to regulate oncogenes and tumor suppressors, and they themselves can act as oncogenes or tumor suppressors. Some miRNAs have been confirmed to promote epithelial-mesenchymal transition (EMT) as well as cancer cell invasion and metastasis. In the case of oncogenic miRNAs, their inhibition can be an effective anti-cancer treatment.

Accordingly, in one aspect, the present application provides a method of producing the compositions of the present application that regulate an activity of a target miRNA to treat a disease or disorder, comprising the following steps: screening one or more of the disclosed compositions that bind with the target miRNA precursor; and analyzing results by a target RNA binding assay disclosed herein. In some embodiments, the miRNA regulates an oncogene or a tumor suppressor, or acts as an oncogene or a tumor suppressor. In some embodiments, the disease is cancer. In some embodiments, the cancer is solid tumor.

There are a variety of oncogenic miRNAs that can be therapeutically targeted, including miR-155, miR-17-92, miR-19, miR-21, and miR-10b. miR-155 plays a pathological role in inflammation, hypertension, heart failure, and cancer. In cancer, miR-155 triggers oncogenic cascades and resistance to apoptosis, as well as an increase in cancer cell invasiveness. Altered expression of miR-155 has been described in a variety of cancers, reflecting stage, progression, and treatment outcomes. Cancers reported to have overexpression of miR-155 are breast cancer, thyroid cancer, colon cancer, cervical cancer, and lung cancer. It is reported to play a role in drug resistance in breast cancer. miR-17-92 (also known as Oncomir-1) is a polycistronic 1 kb primary transcript, which includes miR-17, 20a, 18a, 19a, 92-1, and 19b-1. It is activated by MYC. miR-19 alters gene expression and signal transduction pathways in a variety of hematopoietic cells, and it triggers leukemogenesis and lymphomagenesis. It is involved in a wide variety of human solid tumors and blood cancers. miR-21 is an oncogenic miRNA that reduces the expression of a variety of tumor suppressors. It stimulates cancer cell invasion and is associated with a wide variety of human cancers, including breast cancer, ovarian cancer, cervical cancer, colon cancer, lung cancer, liver cancer, brain cancer, esophageal cancer, prostate cancer, pancreatic cancer, and thyroid cancer. Accordingly, in some embodiments of the methods described above, a target miRNA is selected from miR-155, miR-17-92, miR-19, miR-21, or miR-10b. In some embodiments, the disease or disorder is a cancer selected from: breast cancer, ovarian cancer, cervical cancer, thyroid cancer, colon cancer, liver cancer, brain cancer, esophageal cancer, prostate cancer, lung cancer, leukemia, or lymph node cancer. In some embodiments, the cancer is solid tumor.

Beyond oncology, miRNAs also play a role in many other diseases, ancluding cardiovascular diseases and metabolic diseases.

lncRNAs are RNAs with more than 200 bases (nt) that do not encode proteins. They can affect the expression of protein-coding mRNAs at the levels of transcription, splicing, and mRNA decay. A large number of studies have shown that lncRNAs can regulate transcription by recruiting epigenetic regulators that increase or decrease transcription by altering chromatin structure (e.g., Holoch and Moazed, Nature Reviews Genetics 16:71-84, 2015). lncRNAs are associated with human diseases including: cancer, inflammatory diseases, neurological diseases, and cardiovascular diseases (e.g., Presner and Chinnaiyan, Cancer Discovery 1:391-407, 2011; Johnson, Neurobiology of Disease 46:245-254, 2012; Gutscher and Diederichs, RNA Biology 9:703-719, 2012; Kumar et al., PLOS Genetics 9:e1003201, 2013; van de Vondervoort et al., Frontiers in Molecular Neuroscience, 2013; Li et al., Int. J. Mol. Sci. 14:18790-18808, 2013). lncRNAs can be targeted to upregulate or downregulate the expression of specific genes and proteins to obtain therapeutic benefits (e.g., Wahlestedt, Nature Reviews Drug Discovery 12:433-446, 2013; Guil and Esteller, Nature Structural & Molecular Biology 19:1068-1075, 2012). Generally, lncRNAs are expressed at lower levels relative to mRNAs. Many lncRNAs are physically associated with chromatin (Werner et al., Cell Reports 12, 1-10, 2015) and are transcribed in close proximity to protein-coding genes. They often remain physically associated at their transcription sites and act locally in a cis-acting manner to regulate the expression of adjacent mRNAs. Mutations and dysregulation of lncRNAs are associated with human diseases; therefore, there are numerous lncRNAs that can be therapeutic targets. Accordingly, in some embodiments of the methods described above, the target RNA-non-coding RNA is an lncRNA. In some embodiments, the lncRNA is associated with cancer, inflammatory diseases, neurological diseases, or cardiovascular diseases.

lncRNAs regulate the expression of protein-coding genes, acting at a variety of different levels to affect transcription, alternative splicing, and mRNA decay. For example, an lncRNA has been confirmed to bind with the epigenetic regulator PRC2 to promote its recruitment to genes that are subsequently inhibited for transcription through chromatin modification. lncRNAs can form complex structures that mediate their association with various regulatory proteins. The compositions of the present application binding to these lncRNA structures can be used to regulate the expression of genes normally regulated by individual lncRNAs.

One exemplary target lncRNA is HOTAIR, an lncRNA expressed from the HoxC locus on human chromosome 12. Its expression level is relatively low (approximately 100 RNA copies/cell). Unlike many lncRNAs, HOTAIR can act in trans to affect the expression of distal genes. It binds with the epigenetic repressor PRC2 as well as the LSD1/CoREST/REST complex, another inhibitory epigenetic regulator (Tsai et al., Science 329, 689-693, 2010). HOTAIR is a highly structured RNA, with more than 50% of its nucleotides involved in base pairing. It is often dysregulated (frequently upregulated) in various types of diseases such as cancer (Yao et al., Int. J. Mol. Sci. 15:18985-18999, 2014; Deng et al., PLOS One 9:e110059, 2014). Cancer patients with high HOTAIR expression levels have a significantly worse prognosis compared to those with low expression levels. HOTAIR is reported to be involved in the control of apoptosis, proliferation, metastasis, angiogenesis, DNA repair, chemoresistance, and tumor cell metabolism. It is highly expressed in metastatic breast cancer. High expression levels in primary breast tumors are significant predictors of subsequent metastasis and death. HOTAIR has also been reported to be associated with esophageal squamous cell carcinoma, and it is a prognostic factor in colorectal cancer, cervical cancer, gastric cancer, and endometrial cancer. Accordingly, the compositions of the present application binding with HOTAIR are novel anti-cancer drug candidates. Therefore, in some embodiments of the methods described above, the target RNA-non-coding RNA is HOTAIR. In some embodiments, the disease or disorder is breast cancer, esophageal squamous cell carcinoma, colorectal cancer, cervical cancer, gastric cancer, or endometrial cancer.

Another potential cancer target among lncRNAs is MALAT-1 (metastasis-associated lung adenocarcinoma transcript 1), also known as NEAT2 (nuclear-enriched abundant transcript 2) (Gutschner et al., Cancer Research 73:1180-1189, 2013; Brown et al., Nature Structural & Molecular Biology 21:633-640, 2014). It is a highly conserved 7 kb nuclear lncRNA localized in nuclear speckles. It is widely expressed in normal tissues but is upregulated in many cancers. MALAT-1 is a predictive marker for metastatic development in a variety of cancers including lung cancer. It appears to act as a regulator of gene expression, potentially affecting transcription and/or splicing. MALAT-1 knockout mice exhibit no phenotype, suggesting it has limited normal function. However, cancer cells lacking MALAT-1 show impaired migration and form fewer tumors in mouse xenograft tumor models. Antisense oligonucleotides (ASOs) that block MALAT-1 prevented metastasis formation in mice after tumor implantation. Some mouse xenograft tumor model data indicate that MALAT-1 knockdown via ASOs can inhibit both primary tumor growth and metastasis. Accordingly, it is expected that the compositions of the present application targeting MALAT-1 can effectively inhibit tumor growth and metastasis. Therefore, in some embodiments of the methods described above, the target RNA-non-coding RNA is MALAT-1. In some embodiments, the disease or disorder is a cancer in which MALAT-1 is upregulated, such as lung cancer.

In some embodiments, the present application provides a method of treating a disease or disorder mediated by non-coding RNAs (e.g., HOTAIR or MALAT-1), said method comprising the following steps: administering the compositions of the present application to a patient in need thereof. Such compositions are described in detail herein.

### Target RNA - Toxic RNA (Repeat Sequence RNA)

Simple repeat sequences in mRNAs are often associated with human diseases. They are typically (but not exclusively) repeat sequences with three bases, such as CAG ("triplet repeat sequence"). Triplet repeat sequences are abundant in the human genome, and they tend to undergo expansion over several generations. Approximately 40 types of human diseases are associated with the expansion of repeat sequences. A disease caused by triple expansion is referred to as a triplet repeat expansion disease (TRED). Healthy individuals have a variable number of triplet repeat sequences, but there is a threshold beyond which a higher number of repeat sequences leads to a disease. The threshold differs for different disorders. Triplet repeat sequences may be unstable. When a gene is inherited, the number of repeat sequences may increase, and a condition may become more severe or have an earlier onset from one generation to the next. When an individual has a number of repeat sequences within a normal range, it is expected that they will not expand when passed to the next generation. When the number of repeat sequences is in a premutation range (a normal but unstable number of repeats), the repeat sequences may or may not expand when passed to the next generation. Normal individuals carrying a premutation do not have a condition, but are at risk of having children who inherit triplet repeat sequences in a full mutation range and will be affected. TREDs can be autosomal dominant, autosomal recessive, or X-linked. More common triplet repeat sequences expansion disorders are autosomal dominant.

Repeat sequences can be in the coding or non-coding parts of an mRNA. In cases where the repeat sequences are within non-coding regions, the repeat sequences can be located in 5'UTR, intron, or 3'UTR sequences.

Toxicity produced by the repeat sequences can be a direct result of the action of a toxic RNA itself, or in cases where the repeat sequence expansion is in a coding sequence, due to the toxicity of the RNA and/or an abnormal protein. The repeat sequence expansion RNAs can act by sequestering key RNA-binding proteins (RBPs) into foci. An example of a sequestered RBP is the muscleblind family protein MBNL1. The sequestration of RBPs leads to splicing defects and defects in the nucleo-cytoplasmic transport of RNAs and proteins. The sequestration of RBPs can also affect miRNA biogenesis. These perturbations in RNA biology can greatly affect neuronal function and survival, leading to various neurological diseases.

The present application encompasses a method of treating a disease or disorder, wherein an abnormal RNA itself causes a pathogenic effect, rather than acting through the mechanism of protein expression or the regulation of protein expression. In some embodiments, the disease or disorder is mediated by repeat sequence RNAs. In some embodiments, the disease or disorder is a repeat sequence expansion disease in which the repeat sequences are present in the coding region of an mRNA.

In some embodiments, the present application provides a method of treating a disease or disorder mediated by repeat sequence RNAs, comprising the following steps: administering the compositions of the present application to a patient in need thereof. Such compositions are described in detail herein.

A method of producing a small molecule that regulates an activity of a target repeat sequence expansion RNA to treat a disease or disorder is also provided, comprising the following steps: screening one or more of the disclosed compositions that bind with said target repeat sequence expansion RNA; and analyzing results by an RNA binding assay disclosed herein. In some embodiments, the repeat sequence expansion RNA leads to a disease or disorder selected from: HD, DRPLA, SBMA, SCA1, SCA2, SCA3, SCA6, SCA7, or SCA17. In some embodiments, the disease or disorder is selected from fragile X syndrome, DM1, FRDA, SCA8, SCA10, SCA12, or C9FTD.

### Triple-Helical Polynucleotides

The major groove of a double-stranded RNA (dsRNA) structure can form a triplex with a third strand (triplex-forming oligonucleotide, TFO) through base pairing, which is "triple-helical" base pairing, and it can achieve the inhibition of gene expression.

### Preparation Methods

All polynucleotides of the present application, including antisense molecules, triple-helical DNA, RNA ligands, and ribozymes, can be prepared using any known technique in the art for synthesizing nucleic acid molecules. They include techniques for chemical synthesis of oligonucleotides, such as solid-phase phosphoramidite chemical synthesis. Alternatively, RNA molecules can be prepared by in vitro and in vivo transcription of DNA sequences encoding the polypeptide genes described herein. Such DNA sequences can be incorporated into a variety of vectors having appropriate RNA polymerase promoters (such as T7 or SP6). Alternatively, cDNA constructs that constitutively or inducibly synthesize antisense RNA can be introduced into cell lines, cells, or tissues.

These compositions can be produced by those skilled in the art using routine techniques without undue labor or experimentation. All regions of a gene can be used to design antisense molecules to produce antisense molecules with the strongest hybridization to mRNA, and such suitable antisense oligonucleotides can be prepared and identified by standard assay methods well known to those skilled in the art.

### PNA

The term "peptide nucleic acid" or "PNA" as used herein refers to nucleic acid mimics, such as DNA mimics, in which the deoxyribonucleic acid backbone is replaced with a pseudopeptide backbone, wherein a 2-amino-ethyl-glycine linkage replaces the ribose and phosphodiester backbone, retaining only the four natural nucleobases. It has been shown that the neutral backbone of PNA can specifically hybridize with DNA and RNA under low ionic strength conditions. The synthesis of PNA oligomers can be achieved using standard solid-phase peptide synthesis protocols, as described in Hyrup et al. (1996) and Perry-O'Keefe et al. (1996) Proc. Natl. Acad. Sci. USA 93: 14670-675.

PNA can be used for therapeutic and diagnostic applications. For example, PNA can act as an antisense or antigene agent for sequence-specifically regulating gene expression by, for example, inducing transcriptional or translational arrest or inhibiting replication.

### Modified PNA

The present application provides modified PNAs. In some embodiments, the bases described herein are typically chemically modified on the major groove binding face. Exemplary and non-limiting modifications include amino, mercapto, alkyl, halogen groups, or any modification described herein. The modified bases can be synthesized by any available method as described herein (e.g., by chemical, enzymatic, or recombinant methods to include one or more modified or non-natural bases).

Modified base pairing includes not only standard adenosine-thymine, adenosine-uracil, or guanosine-cytosine base pairs, but also base pairs formed between nucleotides and/or modified nucleotides containing non-standard or modified bases, wherein the arrangement of hydrogen bond donors and hydrogen bond acceptors allows for hydrogen bonding between a non-standard base and a standard base or between two complementary non-standard base structures. An example of such non-standard base pairing is the base pairing between the modified nucleotide inosine and adenine, cytosine, or uracil.

Modified bases and base acids may include modified nucleobases. Examples of nucleobases found in RNA include, but are not limited to, adenine, guanine, cytosine, and uracil. Examples of nucleobases found in DNA include, but are not limited to, adenine, guanine, cytosine, and thymine. These nucleobases can be modified or completely replaced to provide polynucleotide molecules with enhanced properties (e.g., resistance to nucleases, stability), and these properties can be manifested by interfering with the binding of major groove binding partners. For example, the described bases can be chemically modified on the major groove binding face. In some embodiments, the chemical modification of the major groove can include amino, mercapto, alkyl, or halogen groups.

In some embodiments, the peptide nucleic acid comprising the chemical modification are selected from one or more of the peptide nucleic acids as follows:
i) a peptide nucleic acid forming a triplex with a G-C base pair, as shown below; 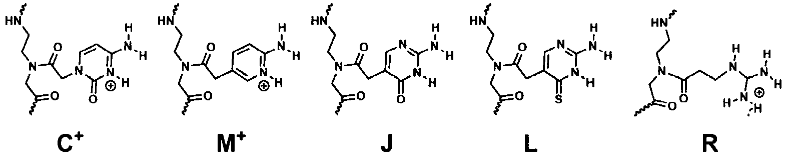 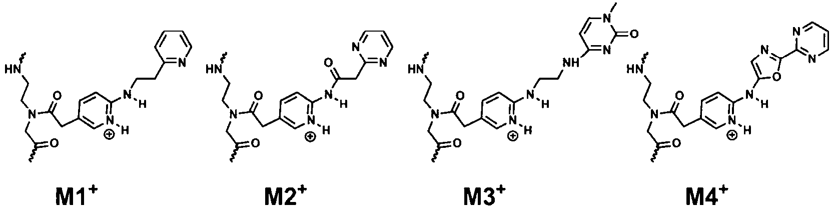
   C⁺: protonated cytosine
   M⁺: protonated 2-aminopyridine
   J: pseudoisocytosine
   L: thiopseudoisocytosine
   R: simple guanidinium moiety
   M1: 2-aminopyridine-1
   M2: 2-aminopyridine-2
   M3: 2-aminopyridine-3
   M4: 2-aminopyridine-4
ii) a peptide nucleic acid forming a triplex with an A-U base pair, as shown below; 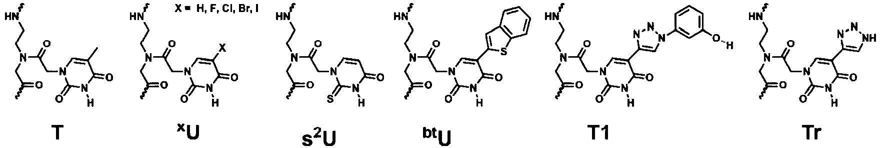
   T: thymine
   ^{x}U: halouracil
   s2U: 2-thiouracil
   ^{bt}U: 5-benzothiopheneuracil
   T1: thymine-1
   Tr: thymine-R
iii) a peptide nucleic acid forming a triplex with a C-G base pair, as shown below; and 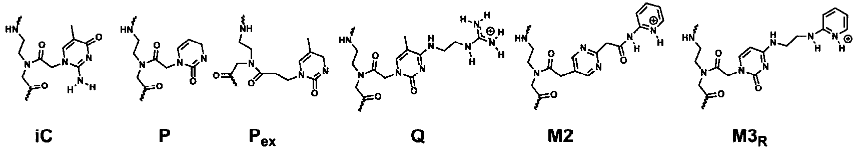
   iC: methylated cytosine
   P: 2-pyrimidinone
   Q: guanidinium-modified cytosine
   M2: 2-aminopyridine-2
   M3R: 2-aminopyridine-3R
iv) a peptide nucleic acid forming a triplex with a U-A base pair, as shown below:

### E: 3-oxo-2,3-dihydropyridine

### S: sulfur

### Hairpin

The term "hairpin" is used to describe a polynucleotide sequence wherein two regions of the same strand are inverse complements of each other in the nucleoside nucleotide sequence, resulting in intramolecular base pairing to form a double-stranded region and an unpaired loop. The term is used herein to describe a DNA sequence encoding such a structure, although typically DNA forms a double strand through intermolecular base pairing. The term is also used to refer to an RNA sequence that adopts a hairpin structure.

The terms "polynucleotide", "oligonucleotide", "nucleic acid", and "nucleic acid molecule" are used interchangeably to refer to a polymeric form of nucleotides of any length, and may include ribonucleotides, deoxyribonucleotides, analogs thereof, or mixtures thereof. The term refers only to the primary structure of the molecule. Thus, the term includes triple-stranded, double-stranded, and single-stranded deoxyribonucleic acid ("DNA"), as well as triple-stranded, double-stranded, and single-stranded ribonucleic acid ("RNA"). It also includes modified, for example by alkylation and/or by capping, as well as unmodified forms of polynucleotides. More specifically, the terms "polynucleotide", "oligonucleotide", "nucleic acid", and "nucleic acid molecule" include polydeoxyribonucleotides (containing 2-deoxy-D-ribose), polyribonucleotides (containing D-ribose), including tRNA, rRNA, hRNA, siRNA, and mRNA, whether spliced or unspliced, any other type of polynucleotide which is an N- or C-glycoside of a purine or pyrimidine base, and other polymers containing non-nucleotidyl backbones, such as polyamides [e.g., peptide nucleic acids ("PNA")] and polymorpholino (commercially available from Anti-Virals, Inc., Corvallis, Oreg., as Neugene) polymers, and other synthetic sequence-specific nucleic acid polymers, provided that the polymer contains nucleobases in a configuration that allows for base pairing and base stacking, such as those found in DNA and RNA.

### Pharmaceutical Compositions

The pharmaceutical compositions disclosed herein are administered to a patient at a therapeutically effective dose, said pharmaceutical compositions being used for preventing, treating, or ameliorating pathological diseases associated with functional changes of RNA elements. A therapeutically effective dose refers to an amount of the compound sufficient to cause substantial inhibition, treatment, or amelioration of the occurrence of said disease. Administration "in combination" with one or more other therapeutic agents includes simultaneous (co-existent) and sequential administration in any order.

The compositions or nucleic acid drugs of the present application comprising a first RNA ligand and a second RNA ligand serve as "active compounds" herein. These compositions or nucleic acid drugs can be incorporated into pharmaceutical compositions suitable for administration to a patient in need thereof.

As used herein, "pharmaceutically acceptable carrier" is intended to include any or all solvents, dispersion media, coatings, antibacterial and antifungal agents, isotonic and absorption delaying agents, and the like, compatible with pharmaceutical administration. Suitable carriers are described in textbooks, such as "Remington's Pharmaceutical Sciences", 20th edition (2000), edited by Gennaro AR, Williams & Wilkins PA, USA, and "Wilson and Gisvold's Textbook of Organic Medicinal and Pharmaceutical Chemistry", Delgado and Remers, Lippincott-Raven, both of which are incorporated herein by reference. Preferred examples of components that can be used in these carriers or diluents include, but are not limited to, water, saline, phosphate, carboxylate, amino acid solutions, Ringer's solution, dextrose solution, and 5% human serum albumin. Liposomes and non-aqueous carriers such as fixed oils can also be used.

The compositions of the present application can be inserted into a vector and used as a gene therapy vector. Gene therapy vectors can include antisense polynucleotides and inhibitory polynucleotides, including microRNA (miRNA), modified miRNA, small inhibitory RNA (siRNA), and modified siRNA, wherein modifications are introduced as described in the present application to at least confer stability to the molecules.

Gene therapy vectors can be delivered to a subject by any of a large number of routes, as described in U.S. Patent No. 5,703,055, being constructed as part of an appropriate nucleic acid expression vector and administered so that they become intracellular, such as by infection using defective or attenuated retroviral or other expression vectors (see, e.g., U.S. Patent No. 4,980,286 and other patents mentioned below) or by direct injection of naked DNA or by use of microparticle bombardment (e.g., gene gun; biolistic, Dupont) or coating with lipids or cell-surface receptors or transfecting agents, encapsulation in liposomes, microparticles, or microcapsules, or by administration in linkage to peptides known to enter the nucleus, by administration in linkage to ligands subject to receptor-mediated endocytosis (see, e.g., U.S. Patent Nos. 5,166,320; 5,728,399; 5,874,297 and 6,030,954, all of which are incorporated herein by reference in their entirety) (which can be used to target specific cell types expressing the receptors), and the like. Thus, delivery can include, for example, intravenous injection, topical administration (see U.S. Patent No. 5,328,470), or stereotaxic injection (see Chen et al. (1994) Proc. Natl. Acad. Sci. USA 91: 3054-3057). Pharmaceutical preparations of gene therapy vectors can include the gene therapy vectors in an acceptable diluent, or can comprise a slow-release matrix in which the gene delivery vectors are embedded. Alternatively, where recombinant cells can produce complete gene delivery vectors (such as retroviral vectors), the pharmaceutical preparations can comprise one or more cells which produce the gene delivery system.

In alternative embodiments, retroviral vectors can be used (see, e.g., U.S. Patent Nos. 5,219,740, 5,604,090, and 5,834,182). These retroviral vectors have been modified to lack unnecessary retroviral sequences for packaging the viral genome and integrating into the host cell DNA. The TORC nucleic acids to be used for gene therapy are cloned into vectors that facilitate the delivery of the genes to a patient.

Adenoviruses are another type of viral vector that can be used in gene therapy. Adenoviruses are particularly advantageous vectors when delivering genes to the respiratory epithelium. Adenoviruses naturally infect the respiratory epithelium, causing mild diseases. Other targets for adenovirus-based delivery systems are the liver, central nervous system, endothelial cells, and muscle. Adenoviruses have the advantage of being able to infect non-dividing cells. Methods for performing adenovirus-based gene therapy are described, for example, in U.S. Patent Nos. 5,824,544; 5,868,040; 5,871,722; 5,880,102; 5,882,877; 5,885,808; 5,932,210; 5,981,225; 5,994,106; 5,994,132; 5,994,134; 6,001,557; and 6,033,8843, all of which are incorporated herein by reference in their entirety.

Adeno-associated virus (AAV) has also been proposed for use in gene therapy. Methods of producing and using AAV are described, for example, in U.S. Patent Nos. 5,173,414; 5,252,479; 5,552,311; 5,658,785; 5,763,416; 5,773,289; 5,843,742; 5,869,040; 5,942,496; and 5,948,675, all of which are incorporated herein by reference in their entirety.

Another method of gene therapy involves the transfer of genes to cells in tissue culture by methods such as electroporation, lipofection, calcium phosphate-mediated transfection, or viral infection. Transfer methods typically include the transfer of a selectable marker into the cells. The cells are then placed under selection pressure to isolate the cells that have taken up and express the transferred gene. These cells are then delivered to a patient. In preferred embodiments, the cells used for gene therapy are autologous cells of the patient.

The dosage and desired pharmaceutical concentration of the pharmaceutical compositions of the present application may vary depending on the specific use. Determining the appropriate dosage or route of administration is within the ability of an ordinary physician. Animal experiments provide reliable guidance for determining the effective dose for human treatment. Interspecies scaling of effective doses can be performed according to the principles specified in Mordenti, J. and Chappell, W. "The use of interspecies scaling in toxicokinetics", Toxicokinetics and New Drug Development, edited by Yacobi et al., Pergamon Press, New York 1989, pages 42-96.

Pharmaceutical compositions suitable for the present application include such compositions wherein an effective dose of the active ingredient is contained to achieve the intended purpose. The determination of an effective dose is within the ability of those skilled in the art.

For any compound, the therapeutically effective dose can be estimated initially in cell culture assays (e.g., cancer cells) or animal models (typically mice, rabbits, dogs, or pigs). Animal models can also be used to determine the appropriate concentration range and route of administration. Dosages can be formulated in animal models to achieve a range of circulating plasma concentrations that includes the IC50 (i.e., the concentration of the test compound that achieves half-maximal inhibition of symptoms). This information can then be used to determine the dosage and route of administration for human administration.

A therapeutically effective dose refers to the amount of the active ingredient effective for preventing, treating, or ameliorating a specific intended pathological disease. Effective doses and toxicity can be determined by standard procedures in cell cultures or experimental animals, such as the ED50 (the dose therapeutically effective in 50% of the population) and the LD50 (the dose lethal to 50% of the population). The dose ratio between toxic effects and therapeutic effects is the therapeutic index, which can be expressed as the ratio LD50/ED50. Pharmaceutical compositions that exhibit high therapeutic indices are preferred. Data obtained from cell culture assays and animal studies are used to formulate the dosage range for use in humans. The dosage contained in these compositions is preferably within a range of circulating concentrations that includes the ED50 with little or no toxicity. The dosage may vary within this range depending upon the dosage form employed, the sensitivity of the patient, and the route of administration.

Specific dosages can be determined by the practitioner considering factors relevant to the subject requiring treatment. The dosage and administration are regulated to provide sufficient levels of the active moiety or to maintain the desired effect. Factors that may be considered include the severity of the disease state, the general health of the subject, the age, weight, and gender of the subject, diet, time and frequency of administration, drug combinations, reaction sensitivity, and tolerance/responsiveness to therapy. Long-acting pharmaceutical compositions may be administered every 3 to 4 days, every week, or once every two weeks, depending on the half-life and clearance rate of the specific formulation.

Depending on the route of administration, normal dosages can vary between 0.1 to 100,000 micrograms, up to approximately 1 g. Guidance regarding specific dosages and delivery methods is provided in the literature and is generally available to practitioners in the art. For bases, those skilled in the art will use dosage forms different from those for proteins or inhibitors thereof. Similarly, the delivery of polybases or polypeptides is to be specifically addressed for specific cells, conditions, locations, etc.

### Therapeutically Effective Amount

As used herein, "therapeutically effective amount" refers to the amount of a drug sufficient to treat or ameliorate the pathological effects of the relevant disorder. For example, a therapeutically effective amount of a composition sufficient to treat or ameliorate the pathological effects of the relevant disorder is the amount sufficient to produce the desired change in the function of the target RNA element.

### Examples

Experimental methods for which specific conditions are not noted in the following examples are performed according to routine methods and conditions, or according to commercial instructions.

The reagents, cell lines, and instruments used in the present application are shown in the table below.

| Reagents | Source | Brand |
|---|---|---|
| High-fidelity polymerase chain reaction kit | NEB | M0494S |
| RNA transcription kit | NEB | E2040S |
| Human embryonic kidney cell line | ATCC | CRL-3216 |
| Lionheart automated live cell imager | Agilent | FX |
| Microplate Reader | PerkinElmer | envision |

### 1. Preparation of the Compositions of the present application

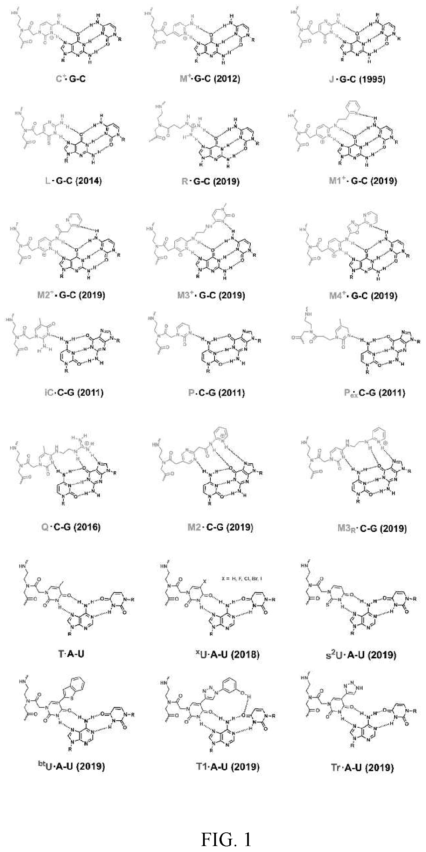

In this example, the composition daPNA targets the double strand-single strand junction region of the target RNA element. The preparation and detection methods comprise the following steps:
1) Designing the daPNA to target and bind the RNA functional element based on dbPNA monomers and asPNA monomers, and linking each monomer to a glycine backbone through solid-phase synthesis to form daPNA oligomers. The dbPNA monomers that can be used are shown in FIG. 1.
2) Purifying via HPLC and identifying by mass spectrometry, and determining the binding affinity between the daPNA and the target RNA through non-denaturing polyacrylamide gel electrophoresis.
3) Using the viral ribosomal frameshifting mechanism as a model, analyzing the biological activity of the daPNA through a cell-free translation system, i.e., the promotion of ribosomal frameshifting efficiency.
4) Delivering the daPNA into cells via liposome delivery, and evaluating the intracellular biological function of the daPNA through dual fluorescent proteins.

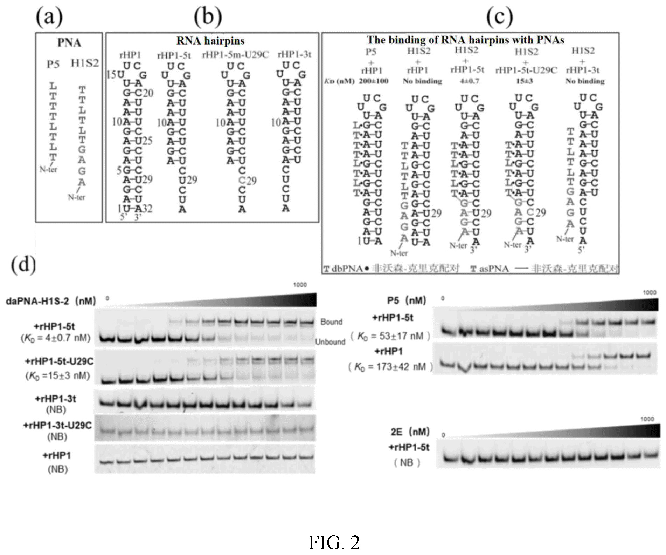

According to the above methods, P5 and H1S1 were designed based on the sequence and hairpin structure of rHP1, as shown in FIG. 2(a). Wherein, the sequence of rHP1 is shown in SEQ ID NO: 1.

rHP1 5'-3' (SEQ ID NO: 1)
UAGAGAAAGUUUCGACUUUCUCUCUCUA

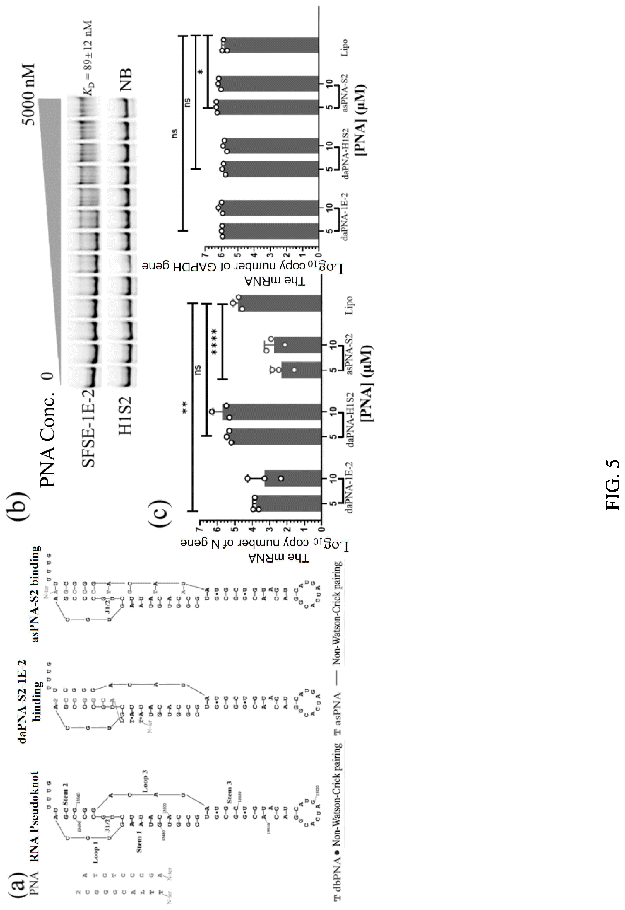

daPNA-SFSE-1E-2 was designed based on the sequence of the SARS-CoV-2 pseudoknot structure (as shown in FIG. 5(a)). Wherein, partial sequences of SARS-CoV-2 are shown in SEQ ID NO: 2-4.
FSE-SARS-CoV-2 (SEQ ID NO: 2) 
UUUAAACGGGUUUGCGGUGUAAGUGCAGCCCGUCUUACACCGUGCGGCACAGGCACU
AGUACUGAUGUCGUAUACAGGGCUUUUGUUUAAACGGGUUUGCGGUGUAAGUGCAGC
CCGUCUUACACCGUGCGGCACAGGCACUAGUACUGAUGUCGUAUACAGGGCUUUUG
5' UTR-SARS-CoV-2 (SEQ ID NO: 3) 
AUUAAAGGUUUAUACCUUCCCAGGUAACAAACCAACCAACUUUCGAUCUCUUGUAGA
UCUGUUCUCUAAACGAACUUUAAAAUCUGUGUGGCUGUCACUCGGCUGCAUGCUUAG
UGCACUCACGCAGUAUAAUUAAUAACUAAUUACUGUCGUUGACAGGACACGAGUAAC
UCGUCUAUCUUCUGCAGGCUGCUUACGGUUUCGUCCGUGUUGCAGCCGAUCAUCAGC
ACAUCUAGGUUUCGUCCGGGUGUGACCGAAAGGUAAG
3' UTR-SARS-CoV-2 (SEQ ID NO: 4)

CAAUCUUUAAUCAGUGUGUAACAUUAGGGAGGACUUGAAAGAGCCACCACAUUUUCA
CCGAGGCCACGCGGAGUACGAUCGAGUGUACAGUGAACAAUGCUAGGGAGAGCUGCC
UAUAUGGAAGAGCCCUAAUGUGUAAAAUUAAUUUUAGUAGUGCUAUCCCCAUGUGAU
UUUAAUAGCUUCUUAGGAGAAUGAC

daPNA-1 was designed based on miRNA-21. Wherein, a partial sequence of miRNA-21 is shown in SEQ ID NO: 5.

UAGCUUAUCAGACUGAUGUUGACUGUUGAAUCUCAUGGCAACACCAGUCGAUGGGCU
GU

The compositions used in the present application are shown in the table below, wherein the first RNA ligand is shown in bold and the second RNA ligand is shown in normal font.

| PNA | **Composition** | **Molecular Formula** | MW |
|---|---|---|---|
| dbPNA-P5 | NH₂-Lys**-TLTLTTTL**-CONH₂ | C₉₁H₁₂₄N₃₈O₂₇S₃ | 2278.41 |
| daPNA-H1S2 | NH₂-Lys-AGAG**TLTLTT-**CONH₂ | C₁₁₄H₁₄₉N₅₇O₃₁S₂ | 2876.12 |
| daPNA-NC | NH₂-Lys-TGTATA**QLL**-CONH₂ | C₁₀₆H₁₄₄N₅₄O₂₇S₂ | 2669.10 |
| daPNA-SFSE-1E-2 | NH₂-Lys-**TTL**ACGGGC2-CONH₂ | C₁₁₂H₁₄₆N₅₈O₃₁S₂ | 2863.1072 |
| daPNA-1 | NH₂-Lys-CAACAG**TQTTQT-**CONH₂ | C₁₄₂H₁₉₃N₇₃O₃₈ | 3528.54 |

P5

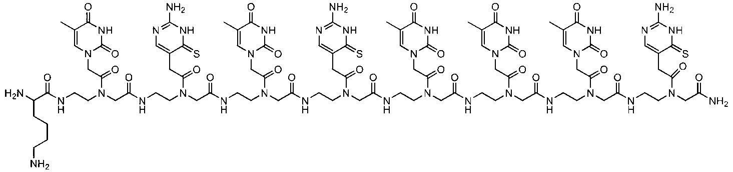

H1S2

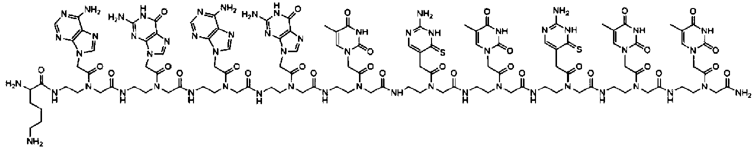

daPNA-NC

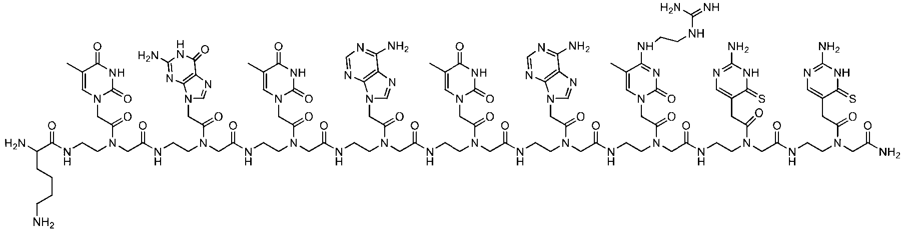

daPNA-SFSE-1E-2

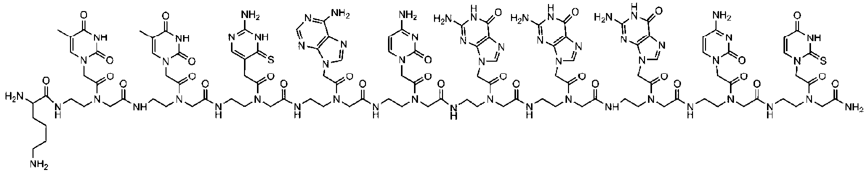

daPNA-1

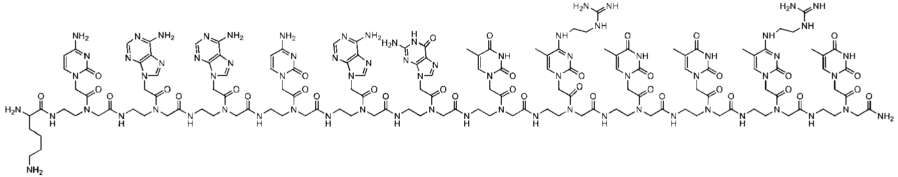

### 2. Activity Assay of daPNA Targeting the rHP1-5t Hairpin Structure

Based on the daPNA-H1S2 designed to bind the rHP1-5t RNA hairpin structure, its stability and its ability to regulate PRF efficiency were determined.

First, the binding affinity between the daPNA-H1S2 and the RNA rHP1-5t was analyzed using polyacrylamide gel electrophoresis, and its K_{D} was approximately 4 ± 0.2 nM. Subsequently, extracellular dual-luciferase and intracellular dual-fluorescent protein assays were respectively employed to verify that the daPNA-H1S2 significantly enhances PRF efficiency.

FIG. 2 illustrates the compositions daPNA H1S2 and dbPNA P5 targets designed based on the RNA hairpin structure of rHP1-5t, as well as the binding results of said compositions with the rHP1-5t hairpin structure and variants thereof;
wherein (a) shows daPNA H1S2 and dbPNA P5 designed based on rHP1-5t; (b) shows the RNA hairpin structure (5t-Cy3); (c) shows the binding of the RNA hairpin to the PNA; and (d) shows non-denaturing PAGE data;
wherein the gel shown is a representative gel; and errors given are standard errors.

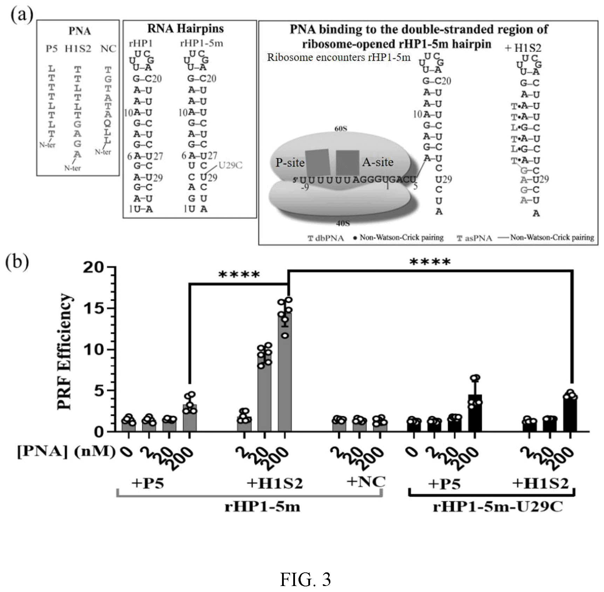

FIG. 3 illustrates the stimulation of PRF efficiency using the composition daPNA H1S2 binding with the rHP1-5t complex;
(a) shows daPNA H1S2, dbPNA P5, and NC designed based on rHP1-5t and their binding to RNA; (b) shows the PRF efficiency of daPNA-H1S2 binding to rHP1-5t. Extracellular luciferase results demonstrated that when the PNA concentration was 200 nM, the daPNA-H1S2 significantly promoted the rHP1-5m frameshifting efficiency to 14.8 ± 1.6%, while the promotion effect on rHPl-5m-U29C was significantly reduced to 4.5 ± 0.2%. In contrast, NC had no effect on the rHP1-5m frameshifting efficiency. P5 promoted rHP1-5m and rHP1-5m-U29C to 3.3 ± 0.9% and 4.5 ± 1.5%, respectively.

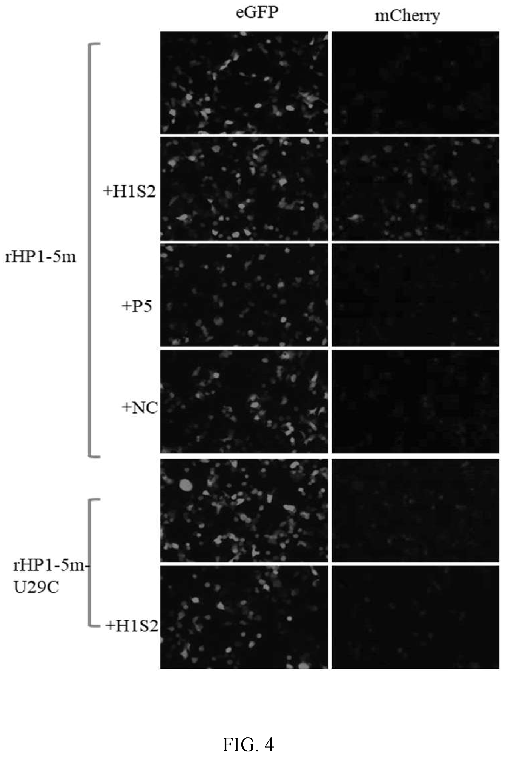

FIG. 4 illustrates the results of mCherry and eGFP fluorescence intensities of a dual-fluorescence protein reporter system using the composition daPNA H1S2 binding with the rHP1-5t complex. The mCherry protein in the dual-fluorescent protein reporter system is the -1 frame encoded protein produced after ribosomal frameshifting occurs. The addition of 10 µM of H1S2 significantly promoted the mCherry expression of rHP1-5m. The expression of H1S2 was able to bind the rHP1-5m mRNA structure intracellularly and promote the ribosomal frameshifting efficiency. In contrast, no significant promotion was observed in the P5 and NC groups. Furthermore, 10 µM H 1S2 was unable to promote the mCherry expression of rHP1 -5m-U29C.

### 3. Activity Assay of daPNA Targeting the SARS-CoV-2 Pseudoknot Structure

The daPNA-SFSE-1E, designed to target the SARS-CoV-2 RNA pseudoknot structure, was able to bind to the corresponding RNA, with a K_{D} value of approximately 80 ± 12 nM. As verified by extracellular luciferase assays, the daPNA-SFSE-1E strongly inhibited the SARS-CoV-2 PRF efficiency. In contrast, the control group showed no significant effect. When the PNA was transfected into ACE2-HEK-293T cells via liposome delivery, it was found to significantly inhibit the replication of the SARS-CoV-2 mRNA.

### 3.1 Regulation of Ribosomal Frameshifting Efficiency by daPNA Extracellularly

A series of daPNAs were designed targeting the SARS-CoV-2 pseudoknot structure to achieve the objective of inhibiting ribosomal frameshifting efficiency by intervening with the formation of the pseudoknot structure.

Based on the results, it was found that daPNA-SFSE-1E-2 had a significant inhibitory effect on the SARS-CoV-2 ribosomal frameshifting.

FIG. 5 illustrates the composition daPNA-SFSE-1E-2 targeting the SARS-CoV-2 pseudoknot structure and the results of its regulation of frameshifting efficiency;
wherein (a) is a schematic diagram of the designed daPNA binding to the SARS-CoV-2 pseudoknot structure; (b) the results of an extracellular regulation of ribosomal frameshifting efficiency by the daPNAs; and (c) an antiviral effect of the composition daPNA-SFSE-1E-2 targeting the SARS-CoV-2 pseudoknot structure;
wherein the mRNA copy number is calculated according to the standard curve formula (Y=-3.7823X+44.339) based on CT values; significance tests are performed in comparison with a liposome control group; ns P > 0.05;* P < 0.1;* * P < 0.01;* * * P < 0.001; daPNA-SFSE-1E-2 can inhibit viral replication in intracellular experiments, with an effective concentration ranging from 0.1 to 20 µM.

### 3.2 daPNA Inhibits SARS-CoV-2 PRF Efficiency and mRNA Replication

The PNA was transfected into ACE2-HEK-293T cells via liposome delivery. Six hours after transfection, the cells were inoculated with SARS-CoV-2 (MOI = 0.1). Forty-eight hours after viral inoculation, the cell supernatant was collected. After total RNA extraction, the mRNA copy number of the SARS-CoV-2 N gene was detected using real-time quantitative PCR (RT-qPCR). The results showed that the daPNA-SFSE-1E-2 was able to significantly inhibit the SARS-CoV-2 replication, wherein the 2E effect was more significant.

FIG. 5(c) illustrates the inhibition of the SARS-CoV-2 PRF efficiency and the antiviral effect of the daPNA-SFSE-1E-2. The mRNA copy number was calculated according to the CT values based on the standard curve formula (Y = -3.7823X + 44.339). Significance testing was performed by comparison with the liposome control group. ns P > 0.05;* P < 0.1;* * P < 0.01;* * * P < 0.001 The daPNA-SFSE-1E-2 was found to inhibit viral replication in intracellular experiments.

### 3.3 daPNA Inhibits the Biological Function of microRNA

A series of daPNAs were designed for the purpose of targeting microRNA (miRNA), and the targeting effects were successfully verified in vivo and in vitro. Using miRNA-21 as an example, the binding of the daPNA to the miRNA-21 precursor was verified in vitro through non-denaturing gel electrophoresis, and the K_{D} value was calculated.

The Cy3-labeled miR-21 precursor (Cy3-pre-miR-21-59nt) was formed into a hairpin structure through snap-cooling, and then slowly cooled from 65°C to room temperature with gradient concentrations of PNA, followed by overnight incubation at 4°C. The samples were subjected to non-denaturing polyacrylamide gel electrophoresis (PAGE) at 400 V for 5 h, showing the binding between the daPNA and the miRNA precursor. The results are shown in FIG. 6.

FIG. 6 illustrates the binding of the daPNA-1 to the miRNA precursor (Cy3-pre-miR-21-59nt) by non-denaturing polyacrylamide gel electrophoresis (PAGE). The results indicated that the daPNA-1 has a high binding affinity for Cy3-pre-miR-21-59nt (A), with a K_{D} value of 0.52 ± 0.05 µM (B).

Incubation buffer: 200 mM NaCl, 0.5 mM EDTA, 20 mM HEPES, pH 7.5. The concentration of Cy3-pre-miR-21-59nt in all samples was 80 nM.

HEK293-T cells were seeded into opaque 96-well plates at a density of 10,000 cells per well. After culturing in an incubator (37°C, 5% CO₂) for 24 h, the full-length miRNA-21 precursor (pre-miR-21-59nt) and gradient concentrations of daPNA-1 were co-transfected into the HEK-293T cells (anti-miR-21 was used as a positive control). After continuing the culture for 24 h, the fluorescence intensity of each well was determined using a microplate reader. Wherein, the ratio of the firefly luciferase (Firefly) intensity to the Renilla luciferase (Renilla) intensity represents the expression level of the target protein under the inhibition of miR-21. The results are shown in FIG. 7.

FIG. 7 illustrates the dual-luciferase assay results of miRNA-21 and daPNA-1, showing that daPNA-1 was able to inhibit the biological function of miRNA-21 in vivo. After miR-21 inhibited the expression level of the target protein to approximately 50% of the normal level, both anti-miR-21 (SEQ ID NO: 6, 5'-ucaacaucagucugauaagcua-3') and daPNA-1 were able to increase the expression level of the target protein by inhibiting the function of miR-21. The inhibitory effect showed a dose-response effect. Specifically, 0.5 µM of daPNA-1 was able to restore the protein expression level to approximately 80%. P-values were calculated using the t-test. A P-value < 0.05 was considered statistically significant.

Furthermore, the miRNA reporter plasmid and the daPNA were transfected into HEK-293T cells via liposome delivery. After 24 h of transfection, the inhibitory effect of the daPNA on the biological function of miRNA-21 intracellularly was verified by measuring the dual-luciferase signals.

The above examples are exemplary and are not to be construed as limitations on the present application. Those of ordinary skill in the art can make changes, modifications, replacements, and variations to the above examples within the scope of the present application. For those of ordinary skill in the art, several improvements and supplementations can also be made without departing from the method of the present application, and such improvements and supplementations shall also be regarded as the protection scope of the present application.

## Claims

1. A composition, **characterized in that** the composition comprises a first RNA ligand and a second RNA ligand; wherein the first RNA ligand comprises an oligonucleotide capable of specifically targeting a double-stranded segment of a target RNA element, and the second RNA ligand comprises an oligonucleotide capable of specifically targeting a single-stranded segment of the target RNA element, such that the composition is capable of specifically targeting a double strand-single strand junction region of the target RNA.

2. The composition according to claim 1, wherein the first RNA ligand comprises an oligonucleotide comprising a chemical modification, wherein the chemical modification results in a selective binding of the first RNA ligand to a double-stranded RNA, and/or
the chemical modification results in an attenuation of binding between the first RNA ligand and single-stranded RNA.

3. The composition according to claim 1 or 2, wherein at least one of the first RNA ligand and the second RNA ligand comprise one or more of a peptide nucleic acid, a ribonucleic acid, a locked nucleic acid, a phosphorodiamidate morpholino oligomer, a glycol nucleic acid, a threose nucleic acid, or a bridged nucleic acid; or,
at least one of the first RNA ligand and the second RNA ligand is connectable to functional molecule having different functions; and
preferably, the functional molecules are one or more selected from a group consisting of a fluorescent molecule, a binding-induced emission molecule, a PET imaging probe, a cell-penetrating small molecule, a targeted delivery molecule, a nanocarrier, an additional RNA ligand, a nanobody, and/or a protein binding ligand.

4. The composition according to any one of claims 1 to 3, wherein the first RNA ligand comprises a chemically modified peptide nucleic acid; and/or
the second RNA ligand comprises an antisense peptide nucleic acid.

5. The composition according to any one of claims 1 to 4, wherein the first RNA ligand comprises no less than 3 bases; and/or,
the second RNA ligand comprises no less than 3 bases; and/or,
the composition comprises no more than 20 bases; and/or
a linker or no linker is included between the first RNA ligand and the second RNA ligand; and
preferably, the linker is one or more aminoacetic acids or diaminobutyric acids.

6. The composition according to any one of claims 1 to 5, wherein the first RNA ligand comprises one or more peptide nucleic acids selected from the following:
i) a peptide nucleic acid forming a triplex with a G-C base pair, as shown below: 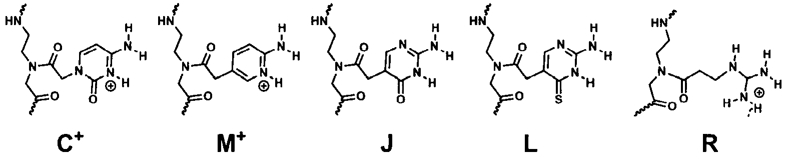 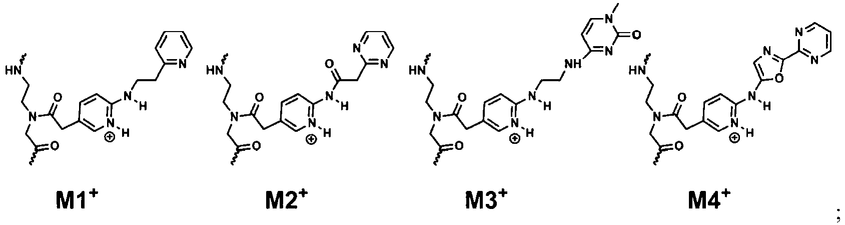
ii) a peptide nucleic acid forming a triplex with an A-U base pair, as shown below: 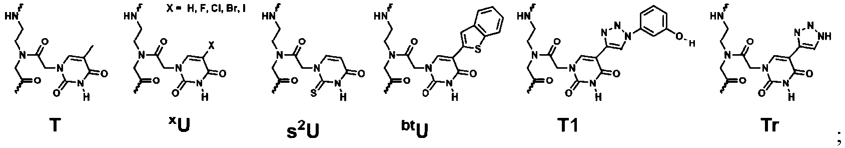
iii) a peptide nucleic acid forming a triplex with a C-G base pair, as shown below: 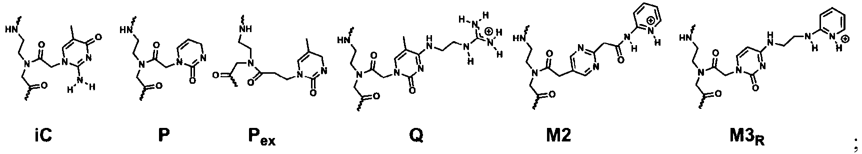 and
iv) a peptide nucleic acid forming a triplex with a U-A base pair, as shown below:

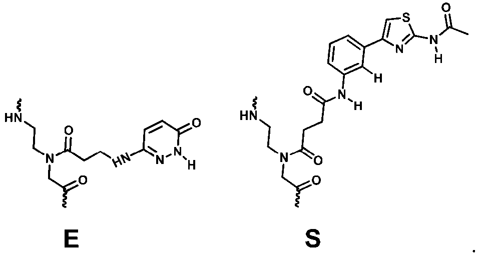

7. The composition according to any one of claims 1 to 6, wherein the target RNA element comprises one or more selected from a group consisting of a nuclear RNA, a cytoplasmic RNA, a membrane RNA, and an extracellular RNA, and the target RNA element comprises a double-stranded segment and a single-stranded segment; and
preferably, the nuclear RNA or the cytoplasmic RNA is selected from a non-coding RNA, an mRNA, a miRNA, an enhancer RNA, a nascent RNA, a chromosome-enriched RNA, a ribosomal RNA, a membrane-enriched RNA, or a mitochondrial RNA.

8. The composition according to claim 7, wherein the target RNA element is associated with a disease or a disorder.

9. The composition according to any one of claims 1 to 8, wherein the target RNA element comprises an RNA sequence selected from a miRNA, a novel coronavirus, or a human immunodeficiency virus HIV;
preferably, the novel coronavirus comprises a pseudoknot structure of a novel coronavirus SARS-CoV-2;
preferably, the miRNA is miRNA-21;
preferably, the composition is a peptide nucleic acid oligomer targeting a double strand-single strand junction region of a pseudoknot structure of a SARS-CoV-2 virus, and has the following structure: 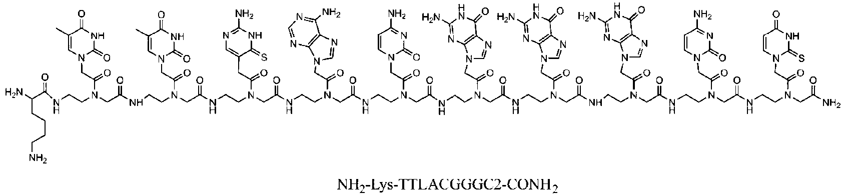 and
preferably, the composition is a composition targeting a double strand-single strand junction region of miRNA-21, and has the following structure: 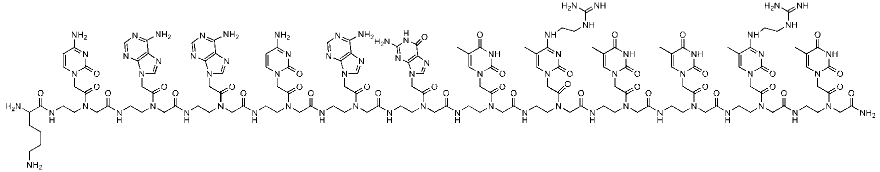 NH₂-Lys-CAACAGTQTTQT-CONH₂.

10. A nucleic acid drug, comprising the composition according to any one of claims 1 to 9.

11. A pharmaceutical composition, comprising the composition according to any one of claims 1 to 9, or the nucleic acid drug according to claim 10, and a pharmaceutically acceptable carrier.

12. Use of the composition according to any one of claims 1 to 9, the nucleic acid drug according to claim 10, or the pharmaceutical composition according to claim 11 in manufacture of a reagent for regulating a function of an RNA element in vivo, wherein:
after the composition, the nucleic acid drug, or the pharmaceutical composition is contacted with an organism, a structure and/or an expression of the RNA element is changed compared to that observed under reference conditions, thereby resulting in a change in a biological function of the RNA element; and
the reference conditions are selected from: an absence of the composition, a presence of a reference composition, and combinations thereof.

13. Use of the composition according to any one of claims 1 to 9, the nucleic acid drug according to claim 10, or the pharmaceutical composition according to claim 11 in manufacture of a medicament for treating a disease,
wherein the disease is associated with a functional change of an RNA element.

14. The use according to claim 13, wherein the disease is selected from a cancer; a neurodegenerative disease; a metabolic disorder; an inflammatory disorder; an autoimmune disease; an infectious disease; or a genetic disease.
